# EUROPEAN PATENT APPLICATION

(11) **EP 3 973 884 A1**
(43) Date of publication of application: **30.03.2022**
(21) Application number: 20834550.4
(22) Date of filing: 19.06.2020
(51) Int. Cl.: A61B 8/00

(54) **DISPLAY DEVICE, DISPLAY METHOD, AND ULTRASONIC DIAGNOSTIC SYSTEM**

(30) Priority: 03.07.2019 JP 2019124201
(71) Applicant: Sony Group Corporation, Tokyo 108-0075 (JP)
(72) Inventor: MAKINO, Takahiro, Tokyo 108-0075 (JP); HAYASHIMOTO, Seiji, Tokyo 108-0075 (JP)
(74) Representative: D Young & Co LLP
(86) International application number: PCT/JP2020/024061
(87) International publication number: WO 2021/002217

(57) **Abstract**

The present technology relates to a display device, a display method, and an ultrasonic diagnostic system capable of providing a realistic ultrasonic diagnostic result.

An image corresponding to an image signal obtained from an output signal of an ultrasonic diagnostic device is displayed on a display panel. Moreover, the display panel is vibrated according to a sound vibration signal obtained from the output signal by an actuator disposed on a back surface side of the display panel, whereby sound that is audibly sensed and vibration that is tactilely sensed are output on the display panel. The present technology can be applied to, for example, the ultrasonic diagnostic system or the like that performs inspection using ultrasonic waves.

## Description

### TECHNICAL FIELD

The present technology relates to a display device, a display method, and an ultrasonic diagnostic system, and particularly relates to, for example, a display device, a display method, and an ultrasonic diagnostic system capable of providing a realistic ultrasonic diagnostic result.

### BACKGROUND ART

For example, as a medical ultrasonic diagnostic system, there is a phonocardiograph that emits ultrasonic waves to an abdomen of a pregnant woman and monitors heart sounds of a fetus in the body of the pregnant woman by a reflection signal of the ultrasonic waves (see, for example, Patent Document 1).

### CITATION LIST

### PATENT DOCUMENT

Patent Document 1: Japanese Patent Application Laid-Open No. 08-154933

### SUMMARY OF THE INVENTION

### PROBLEMS TO BE SOLVED BY THE INVENTION

In recent years, it is required to provide a realistic ultrasonic diagnostic result.

The present technology has been made in view of such a situation, and an object thereof is to provide a realistic ultrasonic diagnostic result.

### SOLUTIONS TO PROBLEMS

A display device of the present technology is a display device including a display panel configured to display an image corresponding to an image signal obtained from an output signal of an ultrasonic diagnostic device, and an actuator disposed on a back surface side of the display panel, and configured to output sound that is audibly sensed and vibration that is tactilely sensed on the display panel by vibrating the display panel according to a sound vibration signal obtained from the output signal.

A display method of the present technology is a display method including displaying an image corresponding to an image signal obtained from an output signal of an ultrasonic diagnostic device, and outputting, by an actuator disposed on a back surface side of the display panel, sound that is audibly sensed and vibration that is tactilely sensed on the display panel by vibrating the display panel according to a sound vibration signal obtained from the output signal.

An ultrasonic diagnostic system of the present technology is an ultrasonic diagnostic system including an ultrasonic probe configured to emit an ultrasonic wave, receive a reflected wave of the ultrasonic wave, and output a reflection signal corresponding to the reflected wave, an ultrasonic diagnostic device configured to generate an output signal by performing signal processing for the reflection signal; and a display device, the display device including a display panel configured to display an image corresponding to an image signal obtained from the output signal of the ultrasonic diagnostic device, and an actuator disposed on a back surface side of the display panel, and configured to output sound that is audibly sensed and vibration that is tactilely sensed on the display panel by vibrating the display panel according to a sound vibration signal obtained from the output signal.

In the display device, the display method, and the ultrasonic diagnostic system of the present technology, the image corresponding to the image signal obtained from the output signal of the ultrasonic diagnostic device is displayed on the display panel. Moreover, the display panel is vibrated according to the sound vibration signal obtained from the output signal by the actuator disposed on the back surface side of the display panel. Therefore, the sound that is audibly sensed and the vibration that is tactilely sensed are output on the display panel.

### BRIEF DESCRIPTION OF DRAWINGS

Fig. 1 is a perspective view illustrating an external configuration example of an embodiment of an ultrasonic diagnostic system to which the present technology is applied.
Fig. 2 is a block diagram illustrating a configuration example of an ultrasonic diagnostic device 11.
Fig. 3 is a block diagram illustrating an electrical configuration example of a display device 12.
Fig. 4 is a perspective view illustrating an outline of an external configuration example of the display device 12.
Fig. 5 is a side view illustrating a configuration example of a side surface of the display device 12.
Fig. 6 is a rear view illustrating a configuration example of a rear surface of the display device 12.
Fig. 7 is a cross-sectional view illustrating a configuration example of a cross section of the display device 12.
Fig. 8 is a cross-sectional view illustrating a configuration example of a cross section of the display device 12.
Fig. 9 is a perspective view illustrating an outline of another external configuration example of the display device 12.
Fig. 10 is a perspective view illustrating an outline of still another external configuration example of the display device 12.
Fig. 11 is a block diagram illustrating another electrical configuration example of the display device 12.
Fig. 12 is a block diagram illustrating a configuration example of another embodiment of an ultrasonic diagnostic system to which the present technology is applied.
Fig. 13 is a block diagram illustrating a configuration example of an embodiment of a computer to which the present technology is applied.

### MODE FOR CARRYING OUT THE INVENTION

### <Embodiment of Ultrasonic Diagnostic System to Which Present Technology is Applied>

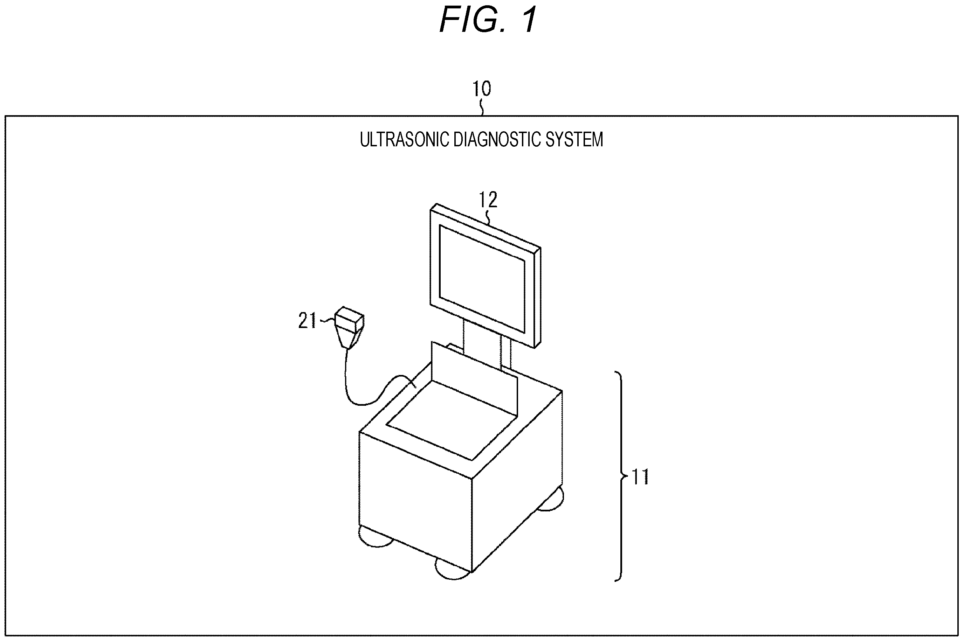

Fig. 1 is a perspective view illustrating an external configuration example of an embodiment of an ultrasonic diagnostic system to which the present technology is applied.

In Fig. 1, an ultrasonic diagnostic system 10 is, for example, a medical ultrasonic diagnostic system for a person as an inspection target, and includes an ultrasonic diagnostic device 11 and a display device 12. Note that the present technology can be applied to an ultrasonic diagnostic system for a living body other than a person or an object other than a living body, such as a building, as an inspection target.

The ultrasonic diagnostic device 11 includes a (ultrasonic) probe 21 and the like, and emits ultrasonic waves from the probe 21. Moreover, the ultrasonic diagnostic device 11 receives reflected waves of the ultrasonic waves reflected and returned by the inspection target by the probe 21, performs signal processing for a reflection signal as an electrical signal corresponding to the reflected waves, thereby generating and outputting an inspection output signal including information of an inside of the inspection target. The inspection output signal includes, for example, an image signal of an image showing an internal state of the inspection target, a sound signal of internal sound of the inspection target, and the like.

The display device 12 receives the inspection output signal output from the ultrasonic diagnostic device 11, and displays an image that is visually sensed (by a person) and outputs sound that is audibly sensed according to the inspection output signal. Moreover, the display device 12 outputs vibration that is tactilely sensed.

Here, in the ultrasonic diagnostic system 10, examples of a method of outputting sound include first, second, and third sound output methods.

The first sound output method is a method of providing a sound output mechanism such as a speaker that outputs sound separately from the display device 12.

The second sound output method is a method of providing a speaker on a rear surface (back surface) of the display device 12 so as to output sound to the rear surface side of the display device 12, and outputting sound from the speaker.

The third sound output method is a method of providing a speaker in a bezel of the display device 12 and outputting sound from the speaker.

In the first sound output method, the sound output mechanism is provided separately from the display device 12, so the volume of the entire ultrasonic diagnostic system 10 becomes large. Therefore, a space required for installing the ultrasonic diagnostic system 10 becomes large, and a place where the ultrasonic diagnostic system 10 is installed may be limited.

In the second sound output method, the speaker is provided so as to output sound to the rear surface side of the display device 12, so a sound volume becomes small, and the sound may be difficult to hear.

In the third sound output method, a hole may be formed in a portion of the bezel where the speaker is provided so that the sound can be clearly heard.

In a medical site where the medical ultrasonic diagnostic system 10 is used, a surface of the display device 12 (display surface (front surface) where an image is displayed) is often contaminated, particularly, contaminated with gel or the like applied to the probe 21, for example. Therefore, cleanability (ease of cleaning) is emphasized on the surface of display device 12.

However, when a hole is formed in the bezel, the cleanability of the display device 12 deteriorates. Moreover, adhesion of contamination to the hole formed in the bezel causes failure of the display device 12. Furthermore, in the case of providing a speaker in the bezel, a certain area or more of the bezel needs to be secured, and the area of the surface of the display device 12 increases. In this case, the space required for installing the ultrasonic diagnostic system 10 becomes large, and the place where the ultrasonic diagnostic system 10 is installed may be limited.

Therefore, in the display device 12, the sound is output by a method other than the first to third sound output methods. Moreover, as described above, the display device 12 displays the image and outputs the sound that is audibly sensed and the vibration that are tactilely sensed, thereby providing a realistic ultrasonic diagnostic result.

### <Electrical Configuration Example of Ultrasonic Diagnostic Device 11>

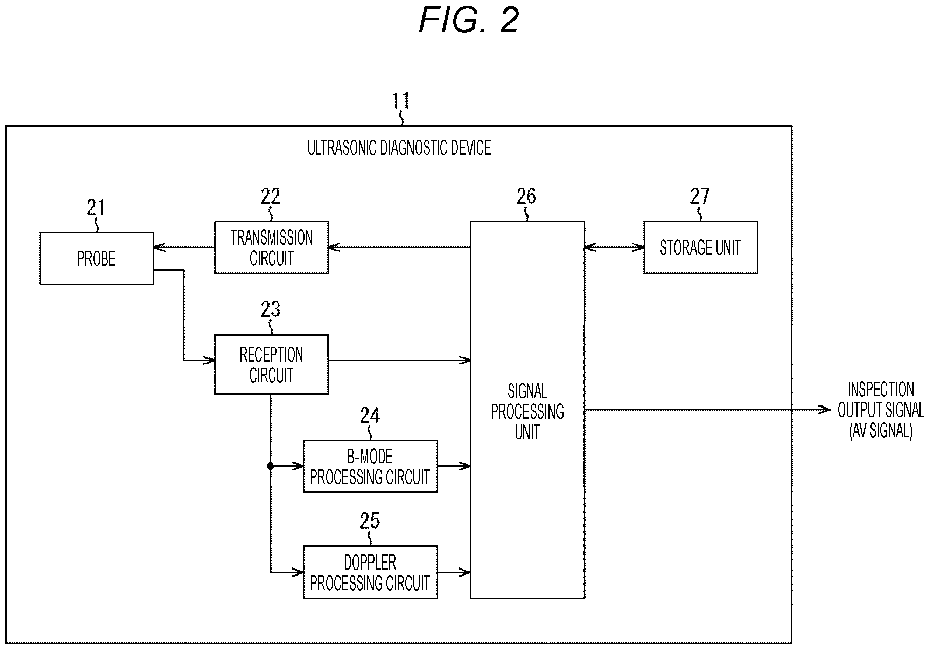

Fig. 2 is a block diagram illustrating a configuration example of the ultrasonic diagnostic device 11 in Fig. 1.

In Fig. 2, the ultrasonic diagnostic device 11 includes a probe 21, a transmission circuit 22, a reception circuit 23, a B-mode processing circuit 24, a Doppler processing circuit 25, a signal processing unit 26, and a storage unit 27.

The probe 21 emits the ultrasonic waves to the inspection target, and receives the reflection signal of the ultrasonic waves reflected by the inspection target. The probe 21 includes a plurality of ultrasonic transducers (not illustrated) such as a plurality of piezoelectric transducers. The plurality of ultrasonic transducers generates the ultrasonic waves in accordance with a drive signal supplied from the transmission circuit 22. The probe 21 focuses the ultrasonic waves generated from the plurality of ultrasonic transducers to transmit a beam-like ultrasonic wave into a body of the inspection target. Moreover, the probe 21 receives the reflected waves of the ultrasonic waves reflected by the inspection target, converts the reflected waves into the reflection signal as an electrical signal, and supplies the reflection signal to the reception circuit 23.

The transmission circuit 22 includes a trigger generation circuit, a transmission delay circuit, a pulser circuit (not illustrated), and the like, and supplies the drive signal to the probe 21. The pulser circuit repeatedly generates rate pulses for forming the ultrasonic waves at a predetermined frequency. The transmission delay circuit gives a delay time for each ultrasonic transducer necessary for focusing the ultrasonic waves generated from the probe 21 in a beam shape and determining transmission directivity to each rate pulse generated by the pulser circuit. The trigger generation circuit applies the drive signal to the probe 21 at timing based on the rate pulse. The transmission circuit 22 changes the delay time given to each rate pulse to arbitrarily adjust a transmission direction from an ultrasonic transducer surface in the transmission delay circuit.

The reception circuit 23 includes an amplifier circuit, an analog to digital (A/D) converter, an adder (not illustrated), and the like. The reception circuit 23 performs various types of processing for the reflection signal from the probe 21 to generate reflection data, and supplies the reflection data to the B-mode processing circuit 24, the Doppler processing circuit 25, and the signal processing unit 26. The amplifier circuit amplifies the reflection signal for each channel and performs gain correction processing. The A/D converter performs A/D conversion for the reflection signal to which the gain correction processing has been applied, and gives a delay time necessary for determining reception directivity to the A/D-converted reflection signal. The adder performs addition processing for the reflection signal processed by the A/D converter to generate the reflection data. By the addition processing of the adder, a reflection component from a direction according to the reception directivity of the reflection signal is emphasized.

The B-mode processing circuit 24 performs logarithmic amplification, envelope detection processing, and the like for the reflection data from the reception circuit 23 to generate B-mode data in which signal intensity is expressed by brightness of luminance, and supplies the B-mode data to the signal processing unit 26.

The Doppler processing circuit 25 performs frequency analysis for the reflection data from the reception circuit 23, extracts blood flow, tissue, and contrast agent echo components due to the Doppler effect, generates Doppler data obtained by extracting mobile body information such as average velocity, dispersion, and power for multiple points, and supplies the Doppler data to the signal processing unit 26.

The signal processing unit 26 generates the inspection output signal including internal image and sound signals of the inspection target according to the reflected waves reflected by the inspection target. The signal processing unit 26 generates an image signal (B-mode image signal) in which the intensity of the reflected waves from the inspection target is represented by luminance from the B-mode data from the B-mode processing circuit 24. Furthermore, the signal processing unit 26 generates an image signal (color Doppler image signal) of an average speed image, a distributed image, a power image, or an image obtained by combining these images representing the moving body information from the Doppler data from the Doppler processing circuit 25. Moreover, the signal processing unit 26 generates the sound signal of the internal sound of the inspection target from the reflection data from the reception circuit 23. The signal processing unit 26 outputs a signal including the image signal and the sound signal as described above. The signal output from the signal processing unit 26 is supplied to the storage unit 27, is output as the inspection output signal to be output from the ultrasonic diagnostic device 11 to the outside, and is supplied to the display device 12 connected to the ultrasonic diagnostic device 11.

The storage unit 27 stores the signal from the signal processing unit 26. Furthermore, the storage unit 27 stores data such as various parameters necessary for the signal processing of the signal processing unit 26.

### <Configuration Example of Display Device 12>

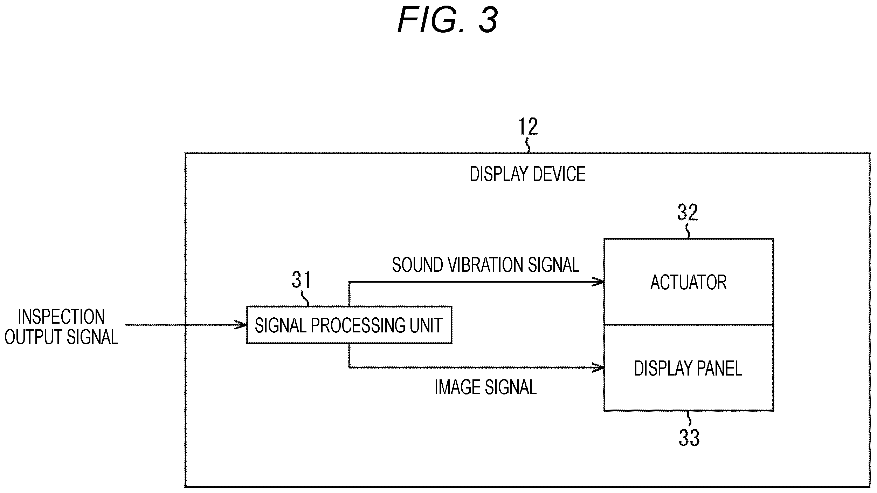

Fig. 3 is a block diagram illustrating an electrical configuration example of a display device 12 in Fig. 1.

In Fig. 3, the display device 12 includes a signal processing unit 31, one or more actuators 32, and a display panel 33.

The inspection output signal output from the ultrasonic diagnostic device 11 is supplied to the signal processing unit 31.

The signal processing unit 31 obtains, from the inspection output signal, the image signal capable of displaying an image on the display panel 33, and a sound vibration signal capable of outputting sound and vibration that can be sensed by a person when the actuator 32 is driven. The image signal is supplied from the signal processing unit 31 to the display panel 33, and the sound vibration signal is supplied from the signal processing unit 31 to the actuator 32.

For example, the signal processing unit 31 extracts the image signal included in the inspection output signal, and supplies the image signal to display panel 33.

Furthermore, for example, the signal processing unit 31 extracts the sound signal included in the inspection output signal. Moreover, for example, the signal processing unit 31 performs image processing or the like using the image signal included in the inspection output signal, thereby recognizing movement of the inside of the inspection target (for example, an organ, a fetus, a blood flow, and the like) shown in the image corresponding to the image signal, and processes the sound signal so as to generate vibration expressing the movement. The signal processing unit 31 supplies the processed sound signal to the actuator 32 as the sound vibration signal for outputting sound and outputting vibration.

As processing of the sound signal, filtering with a band pass filter can be adopted. By filtering (equalizing) the sound signal with a band pass filter and emphasizing a specific frequency band, the frequency of vibration output by driving the actuator 32 can be controlled.

The actuator 32 is disposed on a rear surface of display panel 33. Actuator 32 causes the display panel 33 to output the sound and vibration according to the sound vibration signal from signal processing unit 31.

That is, the actuator 32 is driven according to the sound vibration signal from signal processing unit 31 to vibrate display panel 33. The actuator 32 vibrates the display panel 33 to cause the display panel 33 to output the sound that is audibly sensed and the vibration that is tactilely sensed.

The display panel 33 is a plate-like display panel, and displays the image corresponding to the image signal from the signal processing unit 31. Moreover, the display panel 33 vibrates in response to the driving of the actuator 32, and outputs the sound that is audibly sensed and the vibration that is tactilely sensed.

The sound output from the display panel 33 can be performed by, for example, a technology called an acoustic surface using the display panel as a diaphragm of a flat panel speaker.

As the display panel 33, an organic light emitting diode (OLED) panel or a liquid crystal panel can be adopted. Furthermore, as the display panel 33, for example, a reflective display panel adopted in so-called electronic paper can be adopted.

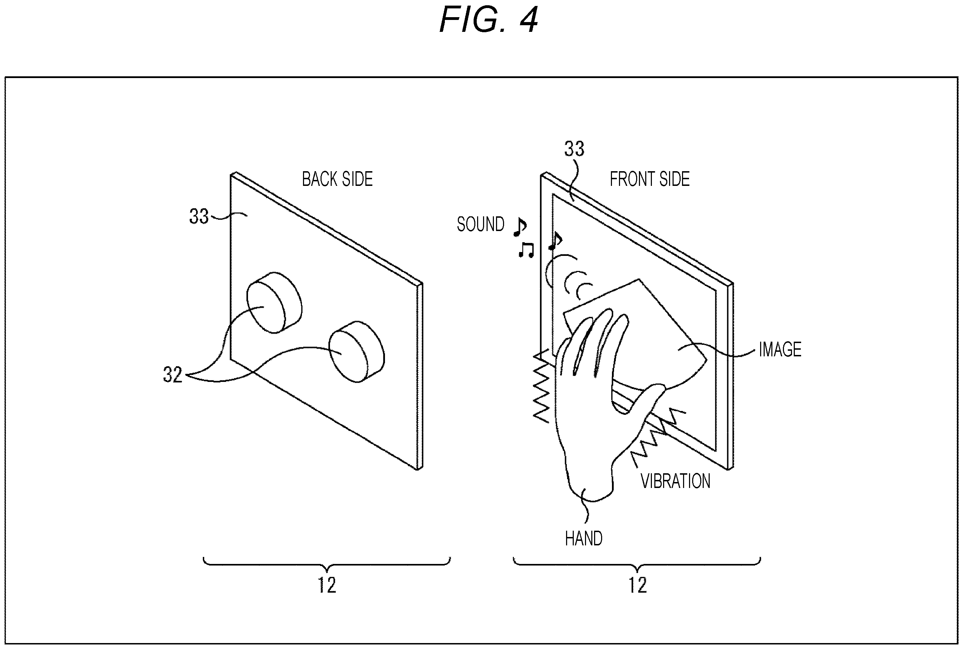

Fig. 4 is a perspective view illustrating an outline of an external configuration example of the display device 12 in Fig. 1.

In the display device 12, the actuator 32 is disposed so as to be in contact with the rear surface (back surface) of plate-like display panel 33. In the display device 12, the actuator 32 vibrates the display panel 33 to cause display panel 33 to output the sound. Therefore, it is not necessary to separately provide a speaker, and the display device 12 can be configured to have a flat structure having no hole formed in the bezel on the surface.

The display device 12 as described above displays the image corresponding to the image signal obtained from the inspection output signal, for example, an image showing the inside of the inspection target. At the same time, the display device 12 outputs the sound and vibration corresponding to the sound vibration signal obtained from the inspection output signal, for example, the internal sound of the inspection target and the vibration expressing the internal movement of the inspection target.

As described above, the display device 12 outputs the image, the sound, and the vibration from the screen at the same time.

Therefore, by touching a screen of the display device 12 while viewing the image of the inside of the inspection target and listening to the sound generated inside the inspection target, the user can feel the vibration expressing the internal movement of the inspection target. As a result, according to the display device 12, a realistic ultrasonic diagnostic result that is visually, audibly, and tactilely sensed at the same time can be provided.

Here, in ultrasonic diagnosis by the medical ultrasonic diagnostic system 10, ultrasonic diagnosis is performed for a person who is a living body as the inspection target. In this case, the display device 12 displays an image related to the living body of the inspection target, for example, an image showing an organ, a fetus, or the like in the living body. Furthermore, the display device 12 outputs sound related to the living body of the inspection target, for example, sound such as biological sound (heartbeat or the like) or a pulse of an organ in the living body. Moreover, the display device 12 outputs vibration related to the living body of the inspection target, for example, vibration such as movement or a pulse of an organ in the living body.

For example, the display device 12 can display an image showing a fetus in the body of a pregnant woman, for example, a three-dimensional (3D) moving image (stereoscopic moving image). At the same time, the display device 12 can output a beating sound of the fetus and can output the vibration of the beating of the fetus. In this case, for example, the pregnant woman who is a subject can see the fetus by the image displayed on the display device 12. Moreover, the pregnant woman can listen to the beating sound of the fetus from a region where the fetus appears on the display device 12. Then, the pregnant woman can tactilely feel the vibration of the beating of the fetus by touching the screen of the display device 12.

As described above, by displaying the moving image of the fetus, outputting the beating sound of the fetus, and outputting the vibration of the beating of the fetus, the entertainment property of the ultrasonic diagnosis for the pregnant woman can be improved.

Furthermore, for example, the display device 12 can display an image showing an organ of a patient, and at the same time, can output biological sound of the organ and output vibration such as movement of the organ. In this case, the patient, an operator or the like who operates the ultrasonic diagnostic system 10 can observe the organ from the image displayed on the display device 12. Moreover, the patient, the operator, and the like can listen to the biological sound of the organ from the region where the organ of the display device 12 is reflected. Then, by touching the screen of the display device 12, the patient, the operator, or the like can tactilely feel the vibration such as movement of the organ.

As the vibration output from the display device 12, for example, periodic vibration that expresses a pulse, movement of an organ, or the like (heartbeat or the like) can be adopted. Furthermore, as the vibration to be output from the display device 12, vibration that can be easily sensed by a person, for example, vibration having a frequency (number of vibration) in the range of 20 to 1000 Hz can be adopted. Moreover, the display device 12 can display, for example, a blood flow in different colors according to the direction of the blood flow.

Note that, in Fig. 4, two actuators 32 as a plurality of actuators 32 are provided on the rear surface of the display panel 33, but the number of actuators 32 provided on the display panel 33 is not limited to two and may be one or three or more.

Furthermore, in Fig. 4, the actuators 32 are provided at a position shifted to the left and a position shifted to the right from the center on the rear surface of display panel 33. However, the positions where the actuators 32 are provided on the display panel 33 are not limited to the positions illustrated in Fig. 4.

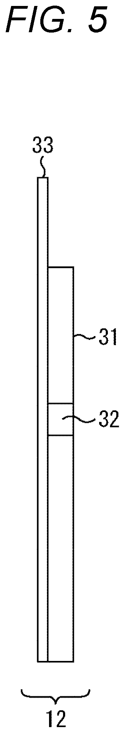

Fig. 5 is a side view illustrating a configuration example of a side surface of the display device 12.

As described with reference to Fig. 3, the display device 12 includes the signal processing unit 31, the actuator 32, and the display panel 33.

The signal processing unit 31 and the actuator 32 are disposed on the rear surface of the display panel 33. The display panel 33 displays the image corresponding to the image signal supplied from the signal processing unit 31, but also functions as a diaphragm vibrated by the actuator 32.

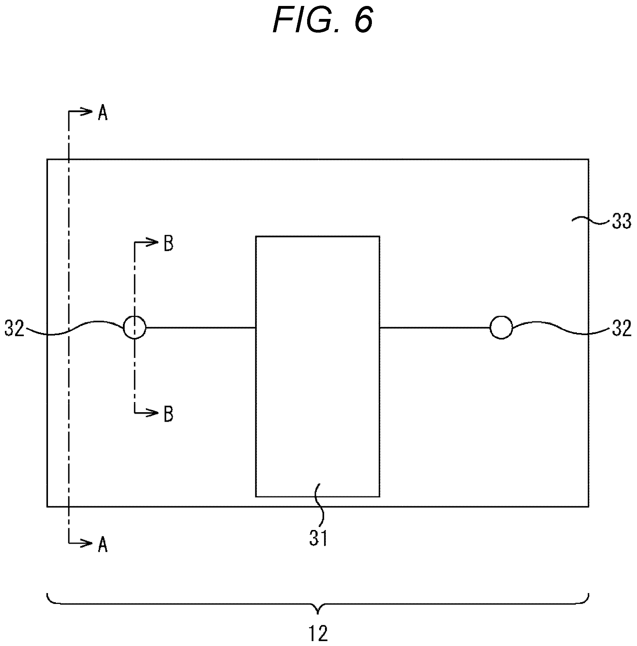

Fig. 6 is a rear view illustrating a configuration example of a rear surface of the display device 12.

In the display device 12, the signal processing unit 31 is disposed, for example, at the center of the rear surface of the display panel 33. For example, the actuator 32 is disposed at the position shifted to the left and the position shifted to the right from the center on the rear surface of the display panel 33.

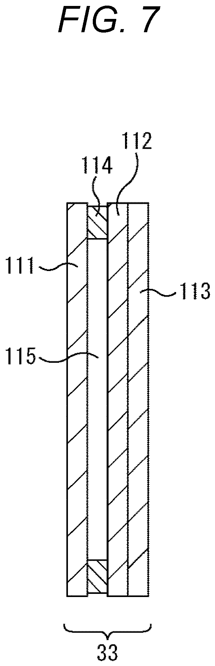

Fig. 7 is a cross-sectional view illustrating a configuration example of a cross section taken along line A-A of the display device 12 in Fig. 6.

Fig. 7 illustrates a configuration example of a cross section of a portion of the display device 12 where the actuator 32 is not provided.

The display panel 33 of the display device 12 includes, for example, a plate-like display cell 111 and an inner plate 112 (counter plate) disposed to face the display cell 111 with a gap 115 interposed therebetween. The display cell 111 displays the image corresponding to the image signal and functions as a diaphragm.

The display panel 33 further includes, for example, a glass substrate 113 disposed in contact with the rear surface of the inner plate 112, and a fixing member 114 disposed between the display cell 111 and the inner plate 112.

The fixing member 114 has a function to fix the display cell 111 and the inner plate 112 to each other and a function as a spacer for maintaining the gap 115. The fixing member 114 is disposed, for example, along an outer edge of the display cell 111. The fixing member 114 is configured by, for example, a buffer layer such as sponge having adhesive layers on both surfaces.

The inner plate 112 is a substrate that supports the actuator 32. The glass substrate 113 has higher rigidity than the inner plate 112 and has a role of suppressing bending of the inner plate 112.

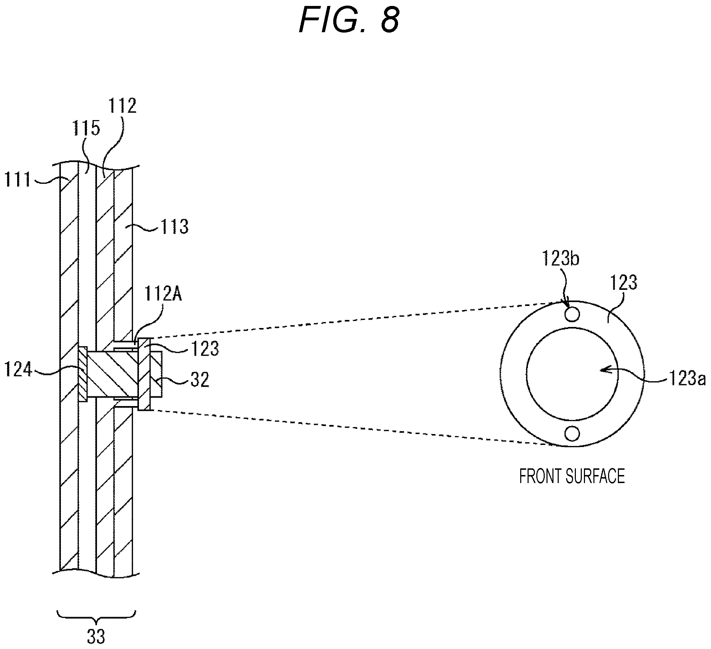

Fig. 8 is a cross-sectional view illustrating a configuration example of a cross section taken along line B-B of the display device 12 in Fig. 6.

Fig. 8 illustrates a configuration example of a cross section of a portion of the display device 12 where the actuator 32 is provided.

Here, the actuator 32 includes, for example, a voice coil, a bobbin around which the voice coil is wound, and a magnetic circuit (all not illustrated). In the actuator 32, when a current flows through the voice coil, a driving force is generated in the voice coil according to the principle of electromagnetic action. Vibration due to the driving force is transmitted to the display cell 111, and the display cell 111 vibrates. When a current corresponding to the sound vibration signal flows through the voice coil of the actuator 32, the display cell 111 vibrates according to the sound vibration signal.

In the display panel 33 of the display device 12, the inner plate 112 has an opening at a position where the actuator 32 is installed, and has a projecting part 112A for supporting a fixing part 123 around the opening. The projecting part 112A projects to an opposite side to the display cell 111. The glass substrate 113 has an opening at a position facing the projecting part 112A. The opening provided in the glass substrate 113 has a size that allows the projecting part 112A and the actuator 32 to be inserted therethrough.

The fixing part 123 has an opening 123a having the actuator 32 inserted therethrough and fixed. The fixing part 123 further includes a plurality of screw holes 123b through which screws used for fixing the fixing part 123 to the projecting part 112A are inserted. The actuator 32 is fixed to the inner plate 112 via the fixing part 123.

A vibration transmission member 124 is in contact with, for example, the back surface of the display cell 111 and the bobbin of the actuator 32, and is fixed to the back surface of the display cell 111 and the bobbin of the actuator 32. The vibration transmission member 124 transmits the vibration due to the driving force generated in the voice coil of the actuator 32 to the display cell 111. The vibration transmission member 124 is configured by, for example, a thermosetting resin, a double-sided tape, or low-repulsion urethane.

### <Another Configuration Example of Display Device 12>

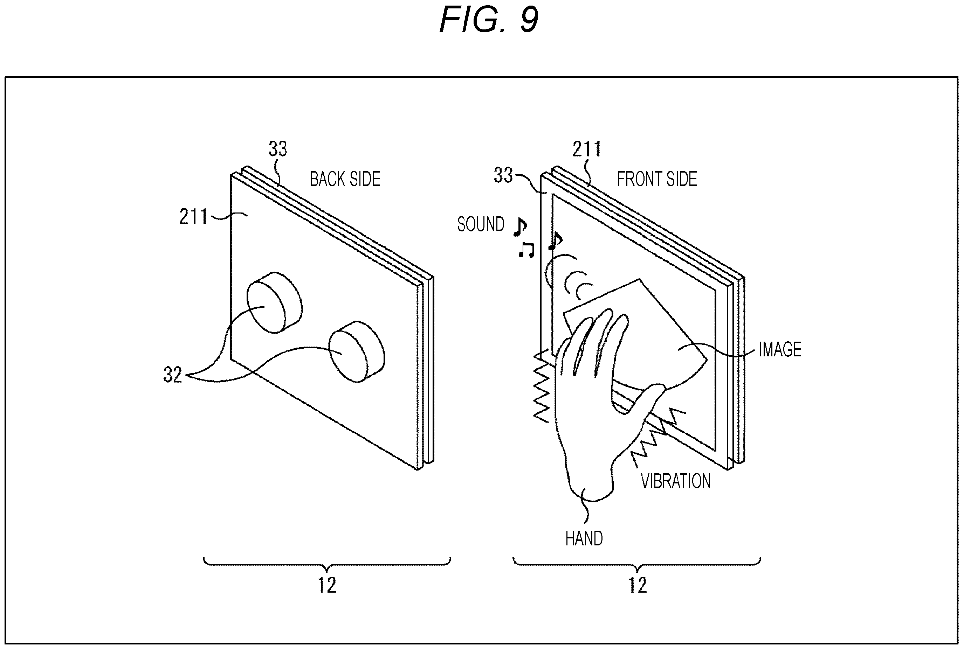

Fig. 9 is a perspective view illustrating an outline of another external configuration example of the display device 12 in Fig. 1.

Note that, in the drawing, parts corresponding to those in Fig. 4 are given the same reference numerals, and hereinafter, description thereof will be omitted as appropriate.

The display device 12 in Fig. 9 is newly provided with a sheet-type heater 211, which is different from the case in Fig. 4 without the sheet-type heater 211.

In the display device 12 in Fig. 9, the thin sheet-type heater 211 is provided on the rear surface of the display panel 33 (display cell 111). The sheet-type heater 211 generates heat according to the control of the signal processing unit 31, for example, and thereby functions as a temperature adjustment unit that adjusts the temperature of the display panel 33.

For example, as described with reference to Fig. 4, even if a pregnant woman can tactilely feel vibration of beating of a fetus by touching the display panel 33 on which a moving image showing the fetus is displayed, the pregnant woman may receive an inorganic impression if she feels cold on the display panel 33 when touching the display panel 33.

Therefore, the display device 12 can raise the temperature of the surface (front surface) of the display panel 33 by the sheet-type heater 211. For example, according to a change in temperature of +3 to +5 degrees, the temperature change can cause a person to feel a living body. Therefore, the sheet-type heater 211 can raise the temperature of the display panel 33 by +3 to +5 degrees (from the current temperature of the display panel 33 or from an ambient temperature).

As described above, by raising the temperature of the display panel 33, for example, the pregnant woman who touches the display panel 33 on which the moving image showing the fetus is displayed can feel realistic feeling as if the pregnant woman actually touches the fetus.

Note that, in Fig. 9, the sheet-type heater 211 is provided on the entire rear surface of the display panel 33, but the sheet-type heater 211 can be provided only on a part of the rear surface of the display panel 33.

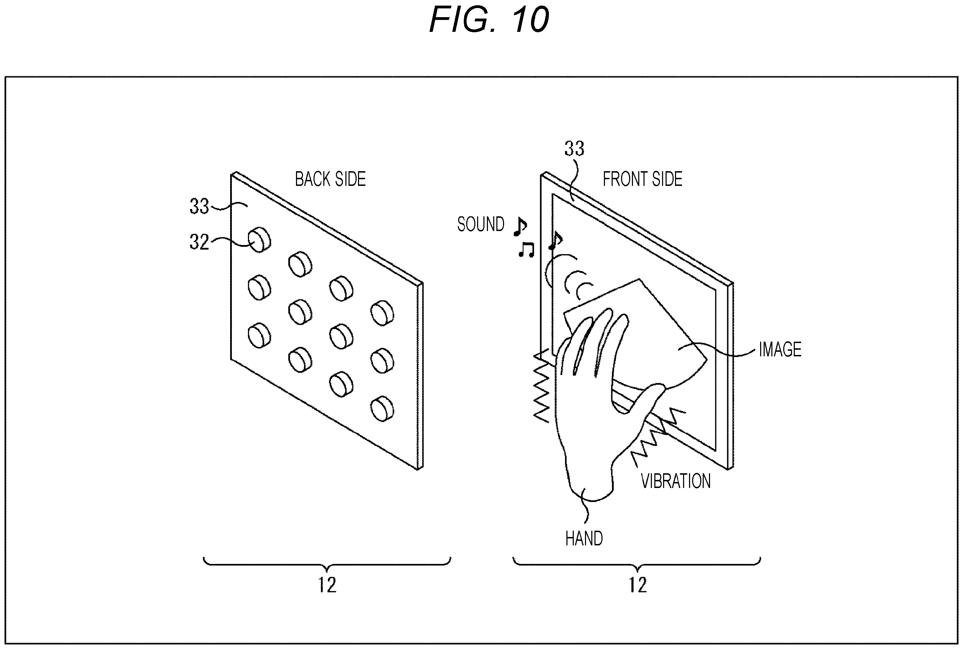

Fig. 10 is a perspective view illustrating an outline of still another external configuration example of the display device 12 in Fig. 1.

Note that, in the drawing, parts corresponding to those in Fig. 4 are given the same reference numerals, and hereinafter, description thereof will be omitted as appropriate.

The display device 12 in Fig. 10 has the number of actuators 32, the number being larger than two, arranged on a two-dimensional plane as the rear surface of the display panel 33, which is different from the case in Fig. 4 in which two actuators 32 are arranged on a straight line on the rear surface of the display panel 33.

In Fig. 10, twelve actuators 32 are arranged on the rear surface of the display panel 33 in a lattice manner.

In this case, the display panel 33 can be divided into small regions centered on the position of the actuator 32, and the intensity of vibration can be controlled for each small region. The intensity of vibration for each small region can be controlled by adjusting (controlling) the sound vibration signal supplied from the signal processing unit 31 to the actuator 32 located at the center of each small region.

As described above, the display panel 33 is divided into the small regions, and the intensity of vibration is controlled for each small region, so that the intensity of the sound and the vibration can be changed for each small region.

By changing the intensity of vibration for each small region, for example, it is possible to output vibration that makes the user feel the direction of blood flow, or to output vibration for each of right atrium, right ventricle, left atrium, and left ventricle of a heart when an image showing the heart is displayed on the display panel 33.

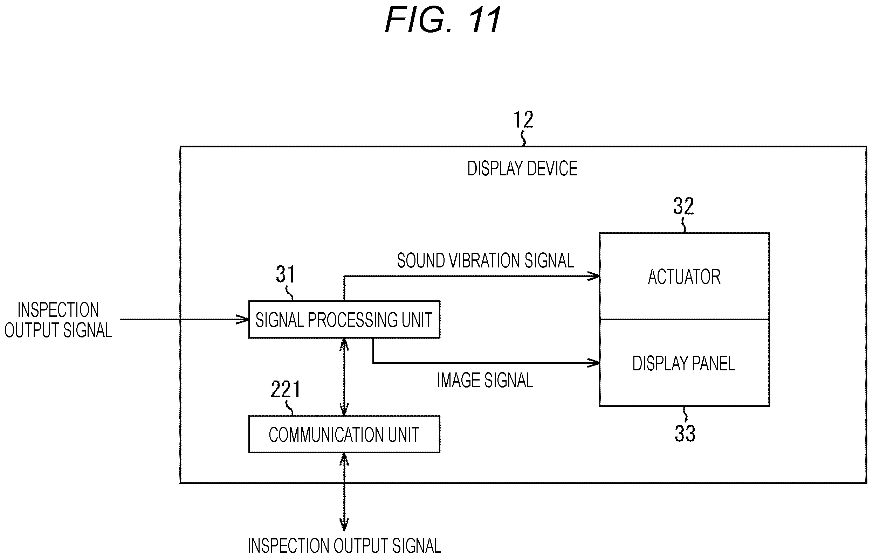

Fig. 11 is a block diagram illustrating another electrical configuration example of the display device 12 in Fig. 1.

Note that, in the drawing, parts corresponding to those in Fig. 3 are given the same reference numerals, and hereinafter, description thereof will be omitted as appropriate.

In Fig. 11, the display device 12 includes the signal processing unit 31 to the display panel 33, and a communication unit 221.

Therefore, the display device 12 in Fig. 11 includes the signal processing unit 31 to the display panel 33, which is common to the case in Fig. 3, and is newly provided with the communication unit 221, which is different from the case in Fig. 3.

The communication unit 221 performs wireless communication with an external device, for example, the ultrasonic diagnostic device 11 or another display device configured similarly to the display device 12.

For example, the communication unit 221 can receive the inspection output signal wirelessly transmitted from the ultrasonic diagnostic device 11 or another display device, and supply the inspection output signal to the signal processing unit 31.

Furthermore, the inspection output signal from the ultrasonic diagnostic device 11 can be supplied from the signal processing unit 31 to the communication unit 221. The communication unit 221 can wirelessly transmit the inspection output signal from the signal processing unit 31 to another display device.

Similarly to the case in Fig. 3, the display device 12 in Fig. 11 can output the image, the sound, and the vibration at the same time according to the inspection output signal (directly) supplied from the ultrasonic diagnostic device 11 to the signal processing unit 31, and can output the image, the sound, and the vibration at the same time according to the inspection output signal transmitted from another display device and supplied to the signal processing unit 31.

### <Another Embodiment of Ultrasonic Diagnostic System to Which Present Technology is Applied>

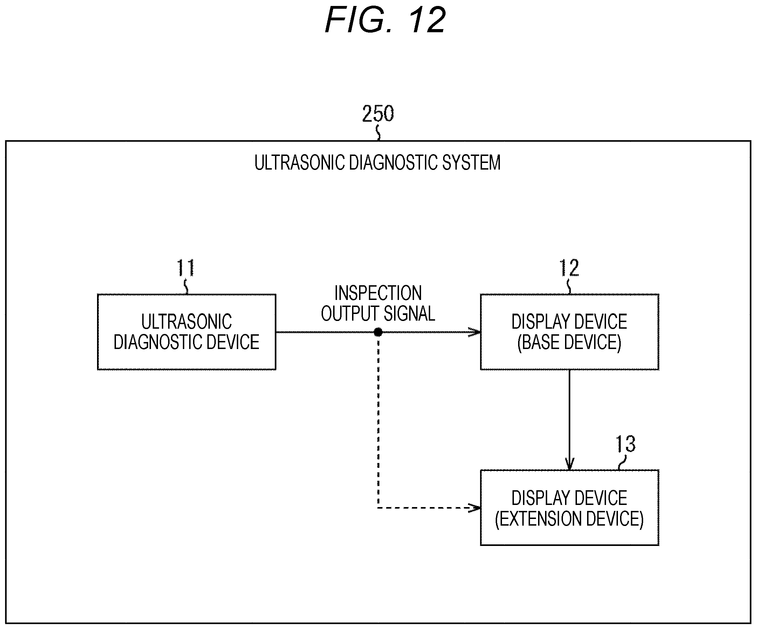

Fig. 12 is a block diagram illustrating a configuration example of another embodiment of an ultrasonic diagnostic system to which the present technology is applied.

Note that, in the drawing, parts corresponding to those in Fig. 1 are given the same reference numerals, and hereinafter, description thereof will be omitted as appropriate.

In Fig. 12, an ultrasonic diagnostic system 250 includes an ultrasonic diagnostic device 11 and display devices 12 and 13.

Therefore, the ultrasonic diagnostic system 250 in Fig. 12 is common to the ultrasonic diagnostic system 10 in Fig. 1 in including the ultrasonic diagnostic device 11 and the display device 12. However, the ultrasonic diagnostic system 250 is different from the ultrasonic diagnostic system 10 in being newly provided with the display device (another display device) 13 different from the display device 12.

In Fig. 12, the display devices 12 and 13 are configured as illustrated in Fig. 11, and have a function to exchange an inspection output signal by wireless communication.

The display device 13 is configured to be portable like a tablet, for example.

In the ultrasonic diagnostic system 250 in Fig. 12, the display device 12 outputs an image, sound, and vibration according to the inspection output signal from the ultrasonic diagnostic device 11.

Moreover, in the ultrasonic diagnostic system 250, when the display device 12 serves as a base device, the portable display device 13 serves as an extension device, and the display device 12 as the base device transmits the inspection output signal from the ultrasonic diagnostic device 11 to the display device 13 as the extension device by wireless communication.

The display device 13 receives the inspection output signal from display device 12, and outputs the image, sound, and vibration according to the inspection output signal, similarly to the display device 12.

As described above, in the ultrasonic diagnostic system 250, the inspection output signal is so-called shared in the display devices 12 and 13, and similar image, sound, and vibration are output according to the inspection output signal.

Here, in the ultrasonic diagnostic system 250, in a case where the display device 12 is housed in a rack or the like together with the ultrasonic diagnostic device 11, for example, when a pregnant woman as an inspection target is undergoing ultrasonic diagnosis, it may be difficult for the pregnant woman to touch the display device 12 on which an image showing a fetus is displayed.

In this case, if the portable display device 13 is handed over to the pregnant woman, the pregnant woman can touch the display device 13 and feel the vibration at the same time as viewing the image and the sound while receiving the ultrasonic diagnosis.

Note that the inspection output signal can be directly supplied from the ultrasonic diagnostic device 11 to the display device 13. Note that, in this case, it is necessary to change the specification of the ultrasonic diagnostic device 11 so that the inspection output signal can be supplied from the ultrasonic diagnostic device 11 to not only the display device 12 but also both the display devices 12 and 13. In contrast, in the case where the inspection output signal supplied from the ultrasonic diagnostic device 11 from the display device 12 is transmitted to the display device 13, it is not necessary to change the specification of the ultrasonic diagnostic device 11.

Furthermore, in Fig. 12, one display device 13 is provided as the extension device, but a plurality of display devices configured similarly to the display device 13 can be provided as the extension devices. For example, by using a display device configured similarly to the display device 13, a family member of the subject can also feel the vibration at the same time as viewing the image and the sound.

As described above, in the display device 12, the image corresponding to the image signal obtained from the inspection output signal of the ultrasonic diagnostic device 11 is displayed on the plate-like display panel 33, and the actuator 32 arranged on the back surface of the display panel 33 vibrates the display panel 33 according to the sound vibration signal obtained from the inspection output signal to output the sound that is audibly sensed and the vibration that is tactilely sensed on the display panel 33. Therefore, a realistic ultrasonic diagnostic result can be provided.

For example, the display device 12 can output the vibration of the beating of the fetus at the same time with the display of the image showing the fetus in the body of the pregnant woman and the output of the beating sound of the fetus. In this case, the pregnant woman can tactilely feel the vibration of the beating of the fetus by viewing the image showing the fetus and the beating sound of the fetus, and touching the screen of the display device 12. Therefore, the pregnant woman can enjoy a realistic sensation as if actually touching the fetus.

Moreover, in the display device 12, the actuator 32 is disposed on the rear surface of the display panel 33. Therefore, the bezel of display device 12 can be reduced and space saving of the entire display device 12 can be achieved.

Furthermore, in display device 12, the actuator 32 vibrates the display panel to output the sound. Therefore, a hole for providing a speaker does not need to be provided in the bezel. Therefore, (the surface of) the display device 12 can be formed flat, and the cleanability can be improved.

Moreover, according to the display device 12, when the display panel 33 vibrates, the sound is output toward the front surface (front) side of the display panel 33. Therefore, the sound can be heard clearly as compared with a case where the speaker is provided so as to output the sound to the rear surface side of the display device 12.

Furthermore, according to the display device 12, since the vibration can be felt at the same time with viewing of the image and the sound, the accuracy of information that can be recognized from the image, the sound, and the vibration is improved, and the accuracy of diagnosis and treatment based on ultrasonic diagnosis at a medical site can be improved.

Moreover, in the display device 12, by raising the temperature of the display panel 33 by about 3 to 5 degrees, the pregnant woman, who views the image displayed on the display panel 33 and the sound output from the display panel 33 and feels the vibration output from the display panel 33, can strongly feel the sense of living body and can feel reality.

Furthermore, in the case of adopting the frequency in the range of 20 to 1000 Hz as the frequency of the vibration output from display panel 33, the vibration easily sensed by a person can be provided, and the person can accurately feel the vibration.

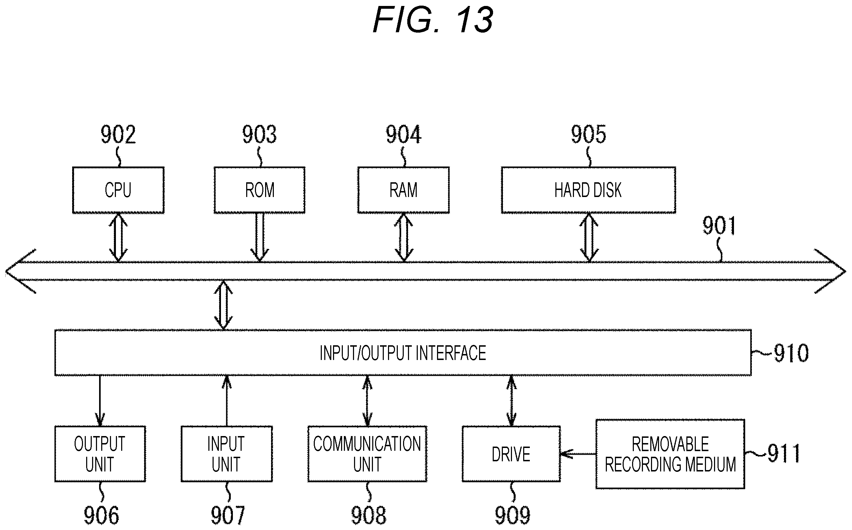

Moreover, as described with reference to Figs. 12 and 13, the display devices 12 and 13 are equipped with the function to exchange the inspection output signal by wireless communication, the display device 13 is configured to be portable like a tablet, and the inspection output signal is transmitted from the display device 12 to the display device 13, whereby the vibration can be felt at the same time as viewing of the image and the sound, using the display device 13, even at a position away from the ultrasonic diagnostic device 11.

Furthermore, as described with reference to Fig. 11, by dividing the display panel 33 into the small regions and arranging the actuator 32 in each small region, the biological sound of a predetermined organ and the vibration such as the movement of the predetermined organ can be output from the region where the predetermined organ of the display panel 33 is shown.

Moreover, by controlling the intensity of the vibration by the actuator 32 in each small region, the user can feel the direction of the blood flow or the like when touching the display panel 33. Note that information of the direction of the blood flow can be included in the inspection output signal in the ultrasonic diagnostic device 11.

### <Description of Computer to Which Present Technology is Applied>

Next, the above-described series of processing of the signal processing unit 31 can be executed by hardware or software. In a case of executing the series of processing by software, a program that configures the software is installed in a microcomputer or the like.

Fig. 13 is a block diagram illustrating a configuration example of an embodiment of a computer to which a program for executing the above-described series of processing is installed.

The program can be recorded in advance in a hard disk 905 or a ROM 903 as a recording medium built in the computer.

Alternatively, the program can be stored (recorded) in a removable recording medium 911 driven by a drive 909. Such a removable recording medium 911 can be provided as so-called package software. Here, examples of the removable recording medium 911 include a flexible disk, a compact disc read only memory (CD-ROM), a magneto optical (MO) disk, a digital versatile disc (DVD), a magnetic disk, a semiconductor memory, and the like.

Note that the program can be downloaded to the computer via a communication network or a broadcast network and installed in the built-in hard disk 905, in addition to the program being installed from the removable recording medium 911 to the computer, as described above. In other words, the program can be transferred in a wireless manner from a download site to the computer via an artificial satellite for digital satellite broadcasting, or transferred in a wired manner to the computer via a network such as a local area network (LAN) or the Internet, for example.

The computer incorporates a central processing unit (CPU) 902, and an input/output interface 910 is connected to the CPU 902 via a bus 901.

When a command is input through the input/output interface 910 by the user who operates an input unit 907 or the like, the CPU 902 executes the program stored in the read only memory (ROM) 903 according to the command. Alternatively, the CPU 902 loads the program stored in a hard disk 905 into a random access memory (RAM) 904 and executes the program.

As a result, the CPU 902 performs the above-described processing according to the flowchart or the above-described processing of the block diagram. Then, the CPU 902 causes an output unit 906 to output the processing result, a communication unit 908 to transmit the processing result, and the hard disk 905 to record the processing result, via the input/output interface 910 as necessary, for example.

Note that the input unit 907 functions as an interface that inputs data from the outside. The output unit 906 functions as an interface that outputs data to the outside.

Here, in the present specification, the term "system" means a group of a plurality of configuration elements (devices, modules (parts), and the like), and whether or not all the configuration elements are in the same housing is irrelevant. Therefore, a plurality of devices housed in separate housings and connected via a network, and one device that houses a plurality of modules in one housing are both systems.

Note that embodiments of the present technology are not limited to the above-described embodiments, and various modifications can be made without departing from the gist of the present technology.

Furthermore, the effects described in the present specification are merely examples and are not limited, and other effects may be exhibited.

Note that the present technology can adopt the following configurations.

A display device including:
a display panel configured to display an image corresponding to an image signal obtained from an output signal of an ultrasonic diagnostic device; and
an actuator disposed on a back surface side of the display panel, and configured to output sound that is audibly sensed and vibration that is tactilely sensed on the display panel by vibrating the display panel according to a sound vibration signal obtained from the output signal.

The display device according to <1>, in which the image is an image related to a living body.

The display device according to <1> or <2>, in which
the sound is sound related to a living body.

The display device according to any one of <1> to <3>, in which
the vibration is vibration related to a living body.

The display device according to any one of <1> to <4>, in which
the vibration is periodic vibration.

The display device according to any one of <1> to <5>, in which
the vibration is vibration having a frequency in a range of 20 to 1000 Hz.

The display device according to any one of <1> to <6>, further including:
a temperature adjustment unit configured to adjust temperature of the display panel.

The display device according to any one of <1> to <7>, further including:
a communication unit configured to transmit the output signal to another display device.

The display device according to any one of <1> to <8>, in which
the display panel is an organic light emitting diode (OLED) panel or a liquid crystal panel.

The display device according to any one of <1> to <8>, in which
the display panel is a reflective display panel.

The display device according to any one of <1> to <10>, including:
a plurality of the actuators.

A display method including:
displaying an image corresponding to an image signal obtained from an output signal of an ultrasonic diagnostic device; and
outputting, by an actuator disposed on a back surface side of the display panel, sound that is audibly sensed and vibration that is tactilely sensed on the display panel by vibrating the display panel according to a sound vibration signal obtained from the output signal.

An ultrasonic diagnostic system including:
an ultrasonic probe configured to emit an ultrasonic wave, receive a reflected wave of the ultrasonic wave, and output a reflection signal corresponding to the reflected wave;
an ultrasonic diagnostic device configured to generate an output signal by performing signal processing for the reflection signal; and
a display device,
the display device including
a display panel configured to display an image corresponding to an image signal obtained from the output signal of the ultrasonic diagnostic device, and
an actuator disposed on a back surface side of the display panel, and configured to output sound that is audibly sensed and vibration that is tactilely sensed on the display panel by vibrating the display panel according to a sound vibration signal obtained from the output signal.

### REFERENCE SIGNS LIST

- 10: Ultrasonic diagnostic system
- 11: Ultrasonic diagnostic device
- 12, 13: Display device
- 21: Probe
- 22: Transmission circuit
- 23: Reception circuit
- 24: B-mode processing circuit
- 25: Doppler processing circuit
- 26: Signal processing unit
- 27: Storage unit
- 31: Signal processing unit
- 32: Actuator
- 33: Display panel
- 111: Display cell
- 112: Inner plate
- 112A: Projecting part
- 113: Glass substrate
- 114: Fixing member
- 115: Gap
- 123: Fixing part
- 123a: Opening
- 123b: Screw hole
- 124: Vibration transmission member
- 211: Sheet-type heater
- 221: Communication unit
- 901: Bus
- 902: CPU
- 903: ROM
- 904: RAM
- 905: Hard disk
- 906: Output unit
- 907: Input unit
- 908: Communication unit
- 909: Drive
- 910: Input/output interface
- 911: Removable recording medium

## Claims

1. A display device comprising:
a display panel configured to display an image corresponding to an image signal obtained from an output signal of an ultrasonic diagnostic device; and
an actuator disposed on a back surface side of the display panel, and configured to output sound that is audibly sensed and vibration that is tactilely sensed on the display panel by vibrating the display panel according to a sound vibration signal obtained from the output signal.

2. The display device according to claim 1, wherein the image is an image related to a living body.

3. The display device according to claim 1, wherein the sound is sound related to a living body.

4. The display device according to claim 1, wherein
the vibration is vibration related to a living body.

5. The display device according to claim 1, wherein the vibration is periodic vibration.

6. The display device according to claim 1, wherein
the vibration is vibration having a frequency in a range of 20 to 1000 Hz.

7. The display device according to claim 1, further comprising:
a temperature adjustment unit configured to adjust temperature of the display panel.

8. The display device according to claim 1, further comprising:
a communication unit configured to transmit the output signal to another display device.

9. The display device according to claim 1, wherein
the display panel is an organic light emitting diode (OLED) panel or a liquid crystal panel.

10. The display device according to claim 1, wherein the display panel is a reflective display panel.

11. The display device according to claim 1, comprising:
a plurality of the actuators.

12. A display method comprising:
displaying, on a display panel, an image corresponding to an image signal obtained from an output signal of an ultrasonic diagnostic device; and
outputting, by an actuator disposed on a back surface side of the display panel, sound that is audibly sensed and vibration that is tactilely sensed on the display panel by vibrating the display panel according to a sound vibration signal obtained from the output signal.

13. An ultrasonic diagnostic system comprising:
an ultrasonic probe configured to emit an ultrasonic wave, receive a reflected wave of the ultrasonic wave, and output a reflection signal corresponding to the reflected wave;
an ultrasonic diagnostic device configured to generate an output signal by performing signal processing for the reflection signal; and
a display device;
the display device including
a display panel configured to display an image corresponding to an image signal obtained from the output signal of the ultrasonic diagnostic device, and
an actuator disposed on a back surface side of the display panel, and configured to output sound that is audibly sensed and vibration that is tactilely sensed on the display panel by vibrating the display panel according to a sound vibration signal obtained from the output signal.
